(19) Europäisches Patentamt
European Patent Office
Office européen des brevets

(11) Veröffentlichungsnummer: **0 303 915 B1**

(12) # EUROPÄISCHE PATENTSCHRIFT

(45) Veröffentlichungstag der Patentschrift: **26.05.93**

(51) Int. Cl.⁵: **A61K 31/255**, A61K 31/095, A61K 31/10, A61K 31/21

(21) Anmeldenummer: **88112801.1**

(22) Anmeldetag: **05.08.88**

(54) **Retinoide zur Behandlung lichtgeschädigter Haut.**

(30) Priorität: **19.08.87 US 86992**

(43) Veröffentlichungstag der Anmeldung:
**22.02.89 Patentblatt 89/08**

(45) Bekanntmachung des Hinweises auf die Patenterteilung:
**26.05.93 Patentblatt 93/21**

(84) Benannte Vertragsstaaten:
**AT BE CH DE FR GB IT LI LU NL SE**

(56) Entgegenhaltungen:
**EP-A- 0 058 370**
**EP-A- 0 253 393**
**EP-A- 0 266 992**
**US-A- 4 603 146**

(73) Patentinhaber: **F. HOFFMANN-LA ROCHE AG**
**Postfach 3255**
**CH-4002 Basel(CH)**

(72) Erfinder: **Bryce, Graeme Findlay**
**245 North Mountain Avenue**
**Upper Montclair, N.J. 07043(US)**
Erfinder: **Shapiro, Stanley Seymour**
**10 Plymouth Drive**
**Livingston, N.J. 07039(US)**

(74) Vertreter: **Lederer, Franz, Dr. et al**
**Lederer, Keller & Riederer Patentanwälte**
**Lucile-Grahn-Strasse 22**
**W-8000 München 80 (DE)**

EP 0 303 915 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Haut, insbesondere die menschliche, weist ein ausgedehntes Netzwerk von Elastinfasern auf, die für die Aufrechterhaltung ihrer elastischen Eigenschaften verantwortlich sind. Bei übermässigem Aussetzen gegenüber Sonnenlicht wird das elastische Fasersystem hyperplastisch, desorganisiert und reisst letztlich.

Diese als aktinische Elastosis bekannte Erscheinung ist der Hauptgrund für das Faltigwerden, die Verfärbung und Erschlaffung der Haut an den exponierten Körperflächen. Die Haut kann sich in einem gewissen Ausmass selbst reparieren, es ist indessen wünschenswert, über ein Mittel zu verfügen, das die Reparatur dieser vorzeitig gealterten Haut beschleunigen kann.

Es wurde gefunden, dass die UV-B bestrahlte haarlose Maus ein geeignetes Modell für die aktinische Elastosis der Haut darstellt (Kligman et al., J Invest. Dermatol. 78, 181 [1982]). Durch Johnston et al. in J. Invest. Dermatol. 82, 587 (1984) wurde gezeigt, dass UV-B Strahlung, die so eingestellt ist, dass sie Sonneneinstrahlung simuliert, zu einer signifikanten Zunahme an Hautelastin führt, die über den Desmosin-Gehalt gemessen werden kann. Die Menge dieser Aminosäure, die durch Säurehydrolyse von Elastin freigesetzt werden kann, ist dem in der Haut anwesenden Elastin proportional (Uitto et al., Lab. Invest. 49, 1216 [1973]). Es wurde gezeigt, dass die topische Behandlung von bestrahlten Mäusen mit Retinsäure das histologische Erscheinungsbild der Haut normalisiert, wobei die vorher elastotische Dermis das Erscheinungsbild von unbestrahltem Gewebe bietet (Kligman et al., Conn. Tissue Res. 12, 139 [1984], Kligman U.S. Patent Nr. 4,603,146). Dieses Modell kann infolgedessen benutzt werden, die Wirksamkeit von Verbindungen bei der Reparatur von lichtgeschädigter Haut zu bestimmen.

Erfindungsgemäss wurde gefunden, dass Verbindungen der Formel

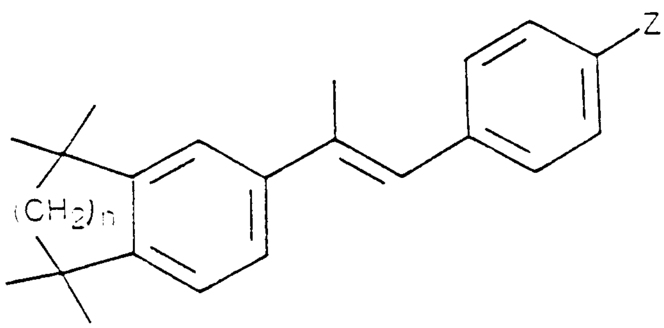

I

worin n 1 oder 2; Z Mercapto oder einen Rest $-S(O)_mR$, m 0, 1 oder 2; R nieder-Alkyl, nieder-Alkenyl, nieder-Alkinyl, nieder-Alkoxy-nieder-alkyl, nieder-Alkanoyl-nieder-alkyl, Hydroxy-nieder-alkyl, Halogen-nieder-alkyl, nieder-Carbalkoxy-nieder-alkyl oder, falls m 1 oder 2 ist, auch nieder-Alkoxy, Hydroxy, mono- oder di-nieder-Alkylamino bedeutet,

und pharmazeutisch verträgliche Salze von Sulfon- und Sulfinsäuren der Formel I mit Ausnahme des Aethyl p-[2-(5,6,7,8-tetrahydro-5,5,8,8-tetramethyl-2-naphthyl)propenyl]phenylsulfons bei der topischen Andwendung auf die Haut eines Patienten die mit Lichtschäden verbundenen Erscheinungen rückgängig machen. Somit kann durch die topische Anwendung von Verbindungen der Formel I auf die Haut von Patienten, die durch Sonnenlicht geschädigt ist, das Faltigwerden, die Elastose und das verfrühte Altern rückgängig gemacht werden, was zu einer Verbesserung des Aussehens der Haut führt.

Durch die topische Verabreichung von Verbindungen der Formel I wird die Reparatur von Hautschäden beschleunigt wodurch die Haut ein glatteres und jüngeres Aussehen erhält.

Der hier verwendete Ausdruck "nieder" bezeichnet Gruppen, die 1-4 C-Atome enthalten. Alkylgruppen können geradkettig oder verzweigt sein. Bevorzugte nieder-Alkylgruppen sind Methyl, Aethyl und Isopropyl. Beispiele von nieder-Alkenylgruppen sind Vinyl, Allyl und Methallyl. Beispiele niederer Alkanoylgruppen sind Acetyl, Propionyl und Butyryl. Alkynylgruppen können geradkettig oder verzweigt sein. Bevorzugte nieder-Alkinylgruppen sind Aethinyl und Propinyl. Der Ausdruck "Halogen" umfasst Fluor, Chlor, Brom und Jod, wobei Chlor bevorzugt ist. Beispiele niederer Carbalkoxy-nieder-alkylgruppen sind Carbomethoxy- und Carboäthoxy-methyl und -äthyl. Beispiele niederer Alkoxygruppen sind Methoxy und Aethoxy. Beispiele von Alkylaminogruppen sind Methylamino, Aethylamino, Isopropylamino, Dimethylamino und Diäthylamino.

Die vorliegende Erfindung betrifft somit die Verwendung von Verbindungen der Formel I und pharmazeutisch verträglichen Salzen davon zur Herstellung von Präparaten für die topische Behandlung von lichtgeschädigter Haut.

Pharmazeutisch anwendbare Salze von Verbindungen der Formel I sind alle Salze, die aufgrund der Chemie der Verbindungen der Formel I möglich sind und die menschlichen Patienten in pharmazeutischen Präparaten verabreicht werden können. Jedes konventionelle, pharmazeutisch anwendbare Salz einer Sulfon- oder Sulfinsäure der Verbindung der Formel I kann angewendet werden. Beispiele solcher Salze sind Alkalimetallsalze wie Natrium- oder Kaliumsalze, Erdalkalimetallsalze wie Calcium- oder Magnesiumsalze, und Ammonium- oder Alkylammoniumsalze. Weiterhin können konventionelle Säureadditionssalze wie Acetate angewandt werden für Verbindungen der Formel I, worin R mono-nieder-Alkylamino oder di-nieder-Alkylamino ist.

Von den Verbindungen der Formel I, in denen Z $-S(O)_mR$darstellt, sind diejenigen Verbindungen bevorzugt, in denen R nieder-Alkyl, nieder-Alkenyl, Hydroxy-nieder-alkyl oder wenn m 1 oder 2 ist, nieder-Alkoxy, Hydroxy, mono-nieder-Alkylamino oder di-nieder-Alkylamino darstellen.

Weiterhin sind diejenigen Verbindungen der Formel I bevorzugt, in denen m = 2 ist und die, in denen R nieder-Alkyl ausgenommen Aethyl ist. Weitere bevorzugte Verbindungen der Formel I sind diejenigen, in denen n 2 ist.

Besonders bevorzugt ist das Methyl p-[2-(5,6,7,8-tetrahydro-5,5,8,8-tetramethyl -2-naphthyl)-propenyl]phenylsulfon.

Andere interessierende Verbindungen der Formel I sind:

Aethyl p-[2-(5,6,7,8-tetrahydro-5,5,8,8-tetramethyl-2-naphthyl))propenyl]phenylsulfonat;
Aethyl p-[2-(5,6,7,8-tetrahydro-5,5,8,8-tetramethyl-2-naphthyl)propenyl]phenylsulfoxid;
Aethyl p-[2-(5,6,7,8-tetrahydro-5,5,8,8-tetramethyl-2-naphthyl)propenyl]phenylsulfid und
Isopropyl p-[2-(5,6,7,8-tetrahydro-5,5,8,8-tetramethyl-2-naphthyl)propenyl]phenylsulfon.

Die Verbindungen der Formel I können wie in der U.S. Patentschrift 4,396,553 beschrieben, hergestellt werden.

Die Verbindungen der Formel I machin wie bereits erwähnt, bei topischer Verabreichung auf die Haut die mit Lichtschäden verbundenen Symptome rückgängig und mässigen und verzögern so die durch Sonne verursachten Hautschäden. Diese Schäden werden mit zunehmendem Alter des Patienten ausgeprägter. Durch topische Verabreichung der Verbindungen der Formel I auf die Haut in einer Menge, die für die Rückgängigmachung der mit Elastosis verbundenen Symptome wirksam ist, wird eine Beschleunigung der Hautreparatur erreicht, wobei die Haut ein glatteres und jüngeres Aussehen erhält. Die Verbindungen der Formel I sollte auf diejenigen Hautgebiete aufgebracht werden, die durch Elastosis beeinträchtigt sind oder deren Behandlung gewünscht wird. Die erfindungsgemässe Verwendung der Verbindungen der Formel I wirkt dem Altern und dem Faltigwerden der Haut entgegen und intensiviert die Wiederherstellung sonnengeschädigter Haut.

Die Verbindungen der Formel I oder eine Kombination davon kann erfindungsgemäss auf der menschlichen Haut in herkömmlichen topischen Zusammensetzungen angewandt werden. Diese Zusammensetzungen können verwendet werden, um die Verbindungen auf die Körperhaut zu bringen, insbesondere auf das Gesicht, die Beine, die Arme und Hände. Zur Erzielung bester Resultate wird die Behandlung begonnen, wenn der Patient ein Alter von zwischen 30 und 55 Jahren hat, die Elastosis zu erscheinen beginnt und ausgeprägter wird. Danach können die Präparate dauernd angewandt werden, um die altersbedingten, mit Elastosis verbundenen Schäden zu reduzieren. Im allgemeinen ist es vorzusiehen, die Behandlung zu beginnen, wenn der Patient ein Alter von etwa 30 Jahren erreicht hat und die Behandlung lebenslang fortzusetzen, um die Auswirkungen der Elastosis zu vermindern und jeden Rückfall zu vermeiden.

Die Verbindungen der Formel I können erfindungsgemäss in jeder herkömmlichen geeigneten topischen Verabreichungsform, d.h. in Kombination mit jedem herkömmlichen Träger, der für die topische Verabreichung geeignet ist, angewandt werden. Die Verbindungen der Formel I können erfindungsgemäss infolgedessen in jeder geeigneten topische Komposition, wie einer Crème, einer Salbe, einer Seife, einer Lösung, einer Lotion, einer Emulsion oder einem Shampoo angewandt werden. Im allgemeinen enthalten diese topischen Präparate zur Erzielung bester Resultate etwa 0,001% bis etwa 1% Gewichtsprozent Verbindung der Formel I, bezogen auf die gesamte Komposition, wobei Mengen von etwa 0,01 bis 1 Gewichtsprozent besonders bevorzugt sind. Gewünschtenfalls können je nach der Art und dem Ausmass der Elastosis höhere Konzentrationen angewandt werden.

Bei der Herstellung dieser Kompositionen können alle konventionellen, nicht toxischen, dermatologisch anwendbaren Trägerstoffe, in denen die Verbindungen der Formel I stabil sind verwendet werden. Bevorzugt für den erfindungsgemässen Zweck sind herkömmliche kosmetische Präparate, die einen weiteren kosmetisch wirksamen Inhaltsstoff enthalten, der bei topischer Verabreichung auf die menschliche Haut einen kosmetischen Effekt ausübt. Solche Stoffe sind Befeuchtungsmittel, oberflächenaktive Stoffe, Weichmacher, Farbstoffe, Conditioner, bakterizide Mittel, Adstringentien und Detergentien.

Die erfindungsgemässen topischen Präparate können gewünschtenfalls geeignete Lichtschutzfilter enthalten. Jedes herkömmliche Lichtschutzmittel kann für den erfindungsgemässen Zweck verwendet werden. Diese topischen, Verbindungen der Formel I enthaltenden Präparate können herkömmliche Excipientien und Zusatzstoffe enthalten, die üblicherweise bei der Herstellung topischer Präparate angewandt werden. Beispiele solcher Stoffe sind Konservierungsmittel, Verdicker und Parfüms. Zusätzlich können herkömmliche Antioxidantien wie butyliertes Hydroxyanisol, Ascorbylpalmitat, Propylgallat, Zitronensäure, butyliertes Hydroxytoluol oder Ethoxyquin diesen Präparaten einverleibt werden. Weiterhin können die topischen Präparate Trägerstoffe enthalten, die im allgemeinen in solchen Präparaten verwendet werden. Die Präparate können übliche Verdickungsmittel, Emulgatoren und Viskositätsstabilisatoren sowie Geschmackstoffe, Farbstoffe und Parfüms enthalten. Die Verbindungen der Formel I enthaltenden topischen Präparate werden vorzugsweise mindestens einmal täglich oder mindestens zwei- bis dreimal wöchentlich auf die Haut aufgebracht. Um die Elastosis rückgängig zu machen und der Haut ein glattes und jüngeres Aussehen zu verleihen sollen die topische Präparate vorzugsweise für die Dauer von mindestens 5 Monate aufgebracht werden. Danach sollten die Präparate weiter angewandt werden, um den Effekt der jüngeren und glatteren Haut aufrechtzuerhalten. Die Präparate können entsprechend den Bedürfnissen des Patienten nach Weisung des Arztes angewandt werden. Jedenfalls wird das Anwendungsschema für die Präparate vom Alter, dem Gewicht und dem Zustand der Haut des betreffenden Patienten abhängen.

Die nachstehenden Beispiele erläutern die Erfindung weiter.

## Beispiel 1

### Behebung UV-B-verursachter Hautschäden bei der haarlosen Maus

Haarlose Mäuse (HRS/J Stamm, Jackson Labs, zu Beginn des Experiments 5-7 Wochen alt) wurden dreimal wöchentlich mit einer Anordnung von 8 Westinhouse Bestrahlungslampen (FS40) die etwa 20 cm oberhalb der Tiere plaziert waren, bestrahlt. Die Strahlungsdosis wurde durch ein handelsübliches phototherapeutisches Kontrollgerät kontrolliert. Die UV-B Dosis wurde so gewählt, dass die Dosierung selten 0,06J/cm$^2$ überschritt, ein minimales Erythem, jedoch keine Verbrennungen oder Narben verursachte. Nach einer Gesamtdosis von etwa 3,5J/cm$^2$ ergab sich eine durch histologischen Befund gesicherte signifikante Elastosis, die durch Messungen des Elastins mittels Radioimmunoassay für Desmosin in der gesamten Haut bestätigt wurde. Der Desmosingehalt erhöhte sich um das zwei- bis dreifache nach 3,5J/cm$^2$ UV-B Bestrahlung. Um die Hautschäden zu beheben, wurde die UV-Bestrahlung unterbrochen und Gruppen der Tiere wurden separat dreimal wöchentlich mit verschiedenen Dosis verschiedener Konzentrationen von Verbindungen der Formel I, in Aceton gelöst, behandelt. Die Lösungen wurde jede Woche frisch so hergestellt, dass die zu verabreichende Dosis in 100 µl Aceton enthalten war und topisch auf ein Gebiet von etwa 10 cm$^2$ auf den Rücken der Tiere aufgebracht. Eine Kontrollgruppe wurde nur mit Aceton behandelt.

Nach 10 Behandlungswochen wurden die Tiere getötet, Hautpräparate angefertigt und durch Luna-Färbung des Elastins das Ausmass der Wiederherstellung quantitativ gemessen. Bei diesem Versuchsmodell wurde die Wiederherstellung der Haut definiert als da Aussehen einer normalen Dermis, die sich von der Epidermis bis auf die Schicht von komprimiertem Elastin erstreckt. Das Ausmass der Wiederherstellung war durch die Breite dieser Zone gegeben. Das Gebiet der Zone auf einer Standardlänge des histologischen Schnitts wurde gemessen und die Resultate als Gesamtfläche in mm$^2$ pro 20 mikroskopische Felder ausgedrückt. Die Resultate sind in Tabelle I zusammengestellt.

### Tabelle I

| | | Reparaturzone, mm$^2$ |
|---|---|---|
| Kontrollwert | | 0.005 +/- 0.001 |
| 0.2µg | Verbindung A | 0.013 +/- 0.002* |
| 0.6 | " | 0.019 +/- 0.005* |
| 2 | " | 0.029 +/- 0.008* |
| 6 | " | 0.007 +/- 0.002 |
| 20 | " | 0.010 +/- 0.003 |

| | |
|---|---|
| Kontrollwert | 0.005 +/- 0.001 |
| 0.1µg Verbindung B | 0.017 +/- 0.007 |
| 0.5 " | 0.021 +/- 0.005* |
| 2 " | 0.027 +/- 0.005** |
| 10 " | 0.028 +/- 0.003*** |
| 50 " | 0.014 +/- 0.005 |

* $P < 0.05$, ** $P < 0.01$, *** $P < 0.001$ vs Kontrollwert

| | |
|---|---|
| Kontrollwert | 0.005 +/- 0.001 |
| 0.1µg Verbindung C | 0.022 +/- 0.007 * |
| 0.5 " | 0.033 +/- 0.007 *** |
| 2 " | 0.023 +/- 0.004 *** |
| 10 " | 0.033 +/- 0.005 *** |

* $P < 0.05$, *** $P < 0.001$ vs Kontrollwert

Verbindung A: Methyl p-[2-(5,6,7,8-tetrahydro -5,5,8,8-tetramethyl-2-naphthyl)-propenyl]-phenylsulfon;

Verbindung B : Methyl p-[2-(5,6,7,8-tetrahydro -5,5,8,8-tetramethyl-2-naphthyl)-propenyl]-phenylsulfoxid;

Verbindung C: Aethyl p-[2-(5,6,7,8-tetrahydro -5,5,8,8-tetramethyl-2-naphthyl)propenyl]-phenylsulfid.

Beispiel 2

Wirkung auf durch UV-B-Bestrahlung verursachte Runzeln bei der haarlosen Maus

Es wurde gefunden, dass Dosen von UV-B-Bestrahlung, die bei der haarlosen Maus Hautschäden verursachen, das Auftreten von Runzeln auf der der Strahlung ausgesetzten Haut verursacht, Von den bestrahlten Gebieten wurden Hautreplikate mit Hilfe einer in der Zahntechnik verwendeten Flüssigkeit angefertigt, um die Hautoberfläche einer visuellen Prüfung zugänglich zu machin. Bei diesen Replikaten erscheinen Runzeln als Vertiefungen, die bei Bestrahlung mit Licht in einem flachen Winkel Schatten werfen. Charakteristisch für das Erscheinungsbild der Runzeln war das Auftreten von Rillen in einer regelmässigen Anordnung mit 2 mm Abstand. Das Ausmass dieser Fältelung wurde unter Verwendung dieses Erscheinungsbilds visuell bestimmt, wobei ein Fältelungsindex von 0 bis 4 zugeordnet wurde. Es wurde beobachtet, dass die Verbindungen der Formel I eine dosisabhängige Glättung der Runzeln verursachte, wobei die $ED_{50}$ im µg-Bereich lag. Die Resultate sind in der nachstehenden Tabelle II zusammengestellt.

## Tabelle II

| | Fältelungsindex |
|---|---|
| Kontrollwert | 1.6 +/- 0.3 |
| 0.2µg Verbindung A | 0.7 +/- 0.3* |
| 2 " | 0.6 +/- 0.3* |
| 6 " | 0.5 +/- 0.1** |
| 20 " | 0.2 +/- 0.1** |
| | |
| Kontrollwert | 1.6 +/- 0.3 |
| 0.1µg Verbindung B | 0.6 +/- 0.2* |
| 0.5 " | 0.3 +/- 0.1** |
| 2 " | 0.7 +/- 0.4 |
| 10 " | 0.2 +/- 0.1** |
| 50 " | 0.4 +/- 0.1** |

* $P < 0.05$, ** $P < 0.01$, *** $P < 0.001$ vs Kontrollwert

Crèmes und Gele mit Inhaltsstoffen in den in Beispiel 3 – 7 angegebenen Proportionen können in üblicher Weise hergestellt werden.

## Beispiel 3

### Crème

| | Gew.-% |
|---|---|
| Verbindung der Formel I | 0.00001-1.3 |
| Cetylalkohol | 1.5 |
| Stearylalkohol | 2.5 |
| Span 60® (Sorbitan-stearat) | 2.0 |
| Mineralöl | 2.0 |
| Arlacel 165 (Glyceryl/PEG 100-stearat) | 4.0 |
| Tween 60® (Polysorbate 80) | 1.0 |
| Miglyol 818 (Capryl-Caprin-/Linolsäure triglycerid) | 5.0 |
| Sorbit-Lösung | 4.0 |
| EDTA-$Na_2$ | 0.1 |
| butyliertes Hydroxyanisol | 0.05 |
| Methylparaben | 0.18 |
| Propylparaben | 0.05 |
| Wasser q.s. | 100.00 |

## Beispiel 4

### Crème

| | Gew.-% |
|---|---|
| Verbindung der Formel I | 0.00001-1.0 |
| Cetylalkohol | 5.25-8.75 |
| Arlacel 165 (Glyceryl/PEG 100-stearat) | 3.75-6.25 |
| Miglyol 818 (Capryl-/Caprin-/Linolsäure triglycerid) | 11.25-18.75 |
| Sorbit-Lösung | 3.75-6.25 |
| EDTA-$Na_2$ | .075-0.125 |

| | |
|---|---|
| Carbopol 934P® (Carbomer 934P) | 0.15-0.25 |
| butyliertes Hydroxyanisol | 0.0375-0.0625 |
| Methylparaben | 0.135-0.225 |
| Propylparaben | 0.0375-0.0625 |
| NaOH (10% solution) | 0.15-0.25 |
| Wasser q.s. | 100.00 |

## Beispiel 5

### Crème

| | Gew.-% |
|---|---|
| Verbindung der Formel I | 0.00001-1.0 |
| Cutina MD (Glyceryl-stearat) | 4.5-7.5 |
| Ceteareth-12 | 3.0-5.0 |
| Cetylalkohol | 3.0-5.0 |
| Generol 122E-10 (Ethoxyliertes Soya-Sterin) | 2.25-3.75 |
| Cetiol LC (Oelsäure-decylester) | 7.5-12.5 |
| butyliertes Hydroxyanisol | 0.0375-0.0625 |
| Sorbit-Lösung | 3.75-6.25 |
| EDTA-$Na_2$ | 0.075-0.125 |
| Methylparaben | 0.135-0.225 |
| Propylparaben | 0.0375-0.0625 |
| Wasser, q.s. | 100.00 |

## Beispiel 6

### Crème

| | Gew.-% |
|---|---|
| Verbindung der Formel I | 0.00001-1.0 |
| Arlatone 983 (PEG 30/Glyceryl-stearat) | 7.0 |
| Cetylalkohol | 1.0 |
| Stearinsäure | 4.0 |
| Neobee Oil (Ttriglycerid mittlerer Kettenlänge) | 17.0 |
| Propylenglycol | 5.0 |

| | |
|---|---|
| 2-Phenoxyäthanol | 0.5 |
| Wasser q.s | 100.00 |

## Beispiel 7

## Gel

| | Gew.-% |
|---|---|
| Verbindung der Formel I | 0.00001-1.0 |
| Pluronic L 101 (Poloxamer 331) | 10.00 |
| Aerosil 200® (Siliciumdioxid) | 8.00 |
| PCL Liquid (Fettsäureester) | 15.00 |
| Cetiol V (Decyloleat) | 20.00 |
| Neobee Oil (Triglycerid mittlerer Kettenlänge) | 15.00 |
| Euhanol G (Octyldodecanol), q.s. | 100.00 |

Durch Variation der Verhältnisse zwischen den Hilfsstoffen der Beispiele 3-7 können die physikalischen Eigenschaften der Präparate verändert werden.

**Patentansprüche**

1. Verwendung von Verbindungen der Formel

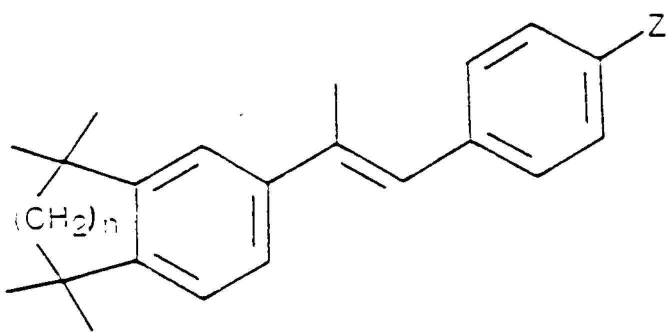

I

worin n 1 oder 2; Z Mercapto oder einen Rest $-S(O)_mR$, m 0, 1 oder 2; R nieder-Alkyl, nieder-Alkenyl, nieder-Alkinyl, nieder-Alkoxy-nieder-alkyl,nieder-Alkanoyl-nieder-alkyl, Hydroxy-nieder-alkyl, Halogen-nieder-alkyl, nieder-Carbalkoxy-nieder-alkoxy oder, falls m 1 oder 2 ist, auch nieder-Alkoxy, Hydroxy, mono- oder di-nieder-Alkylamino bedeutet, wobei "nieder" Gruppen mit 1-4 C-Atomen bezeichnet,
und pharmazeutisch verträglichen Salzenvon Sulfon- und Sulfinsäuren der Formel I mit Ausnahme des Aethyl-p-[2-(5,6,7,8-tetrahydro-5,5,8,8-tetramethyl-2-naphthyl)propenyl]phenylsulfons zur Herstellung von Präparaten für die topische Behandlung von lichtgeschädigter Haut.

2. Verwendung gemäss Anspruch 1 von Verbindungen gemäss Anspruch 1, in denen n = 2, m = 2 und R nieder-Alkyl, ausgenommen Aethyl ist.

**3.** Verwendung gemäss Anspruch 1 von Methyl p-[2-(5,6,7,8-tetrahydro-5,5,8,8-tetramethyl-2-naphthyl)propenyl]phenyl-sulfon.

## Claims

**1.** The use of compounds of the formula

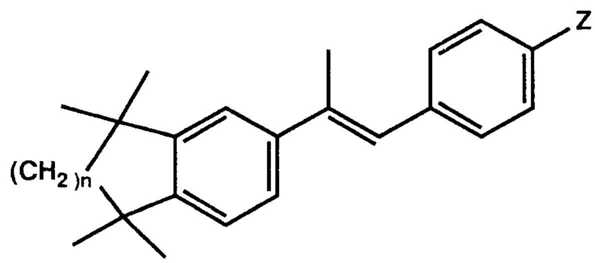

I

wherein n signifies 1 or 2; Z signifies mercapto or a residue $-S(O)_mR$; m signifies 0, 1 or 2; R signifies lower-alkyl, lower-alkenyl, lower-alkynyl, lower-alkoxy-lower alkyl, lower-alkanoyl-lower-alkyl, hydroxy-lower-alkyl, halo-lower-alkyl, lower-carbalkoxy-lower-alkyl or, when m is 1 or 2, R also represents lower-alkoxy, hydroxy, mono-lower-alkylamino or di-lower-alkylamino, whereby "lower" denotes groups with 1-4 C atoms,
and pharmaceutically acceptable salts of sulphonic acids and sulphinic acids of formula I, except ethyl p-[2-(5,6,7,8-tetrahydro-5,5,8,8-tetramethyl-2-naphthyl)propenyl]phenylsulphone, for the manufacture of preparations for the topical treatment of photodamaged skin.

**2.** The use according to claim 1 of compounds in accordance with claim 1 in which n = 2, m = 2 and R is lower-alkyl, except ethyl.

**3.** The use of methyl p-[2-(5,6,7,8-tetrahydro-5,5,8,8-tetramethyl-2-naphthyl)-propenyl]-phenylsulphone according to claim 1.

## Revendications

**1.** Utilisation de composés de formule

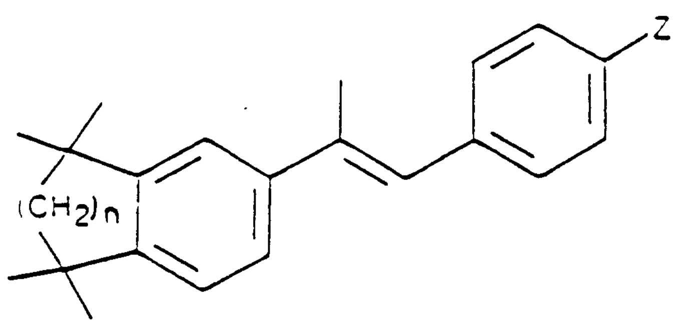

I

dans laquelle n est égal à 1 ou 2 ; Z représente un groupe mercapto ou un groupe $-S(O)_mR$ dans lequel m est égal à 0, 1 ou 2 ; R représente un groupe alkyle inférieur, alcényle inférieur, alcynyle inférieur, (alcoxy inférieur)-alkyle inférieur, (alcanoyle inférieur)-alkyle inférieur, hydroxyalkyle inférieur, halogénoalkyle inférieur, (carbalcoxy inférieur)-alcoxy inférieur, ou bien encore, lorsque m est égal à 1 ou 2, un groupe alcoxy inférieur, hydroxy, mono- ou di-(alkyle inférieur)-amino, l'expression "inférieur" s'appliquant à des groupes en C 1-C 4,
et des sels acceptables pour l'usage pharmaceutique des acides sulfoniques et sulfiniques de formule I, à l'exception de l'éthyl-p-[2-(5,6,7,8-tétrahydro-5,5,8,8-tétraméthyl-2-naphtyl)-propényl]-phénylsulfone, pour la préparation de compositions pour le traitement topique de la peau lésée par la lumière.

2. Utilisation selon la revendication 1 de composés selon la revendication 1, pour lesquels n = 2, m = 2 et R représente un groupe alkyle inférieur à l'exception du groupe éthyle.

3. Utilisation selon la revendication 1, de la méthyl – p – [2 – (5,6,7,8 – tétrahydro – 5,5,8,8 – tétraméthyl – 2 – naphtyl) – propényl] – phénylsulfone.